Europäisches Patentamt

European Patent Office

Office européen des brevets

(19)

(11) Veröffentlichungsnummer: **0 331 806**
**A1**

(12) **EUROPÄISCHE PATENTANMELDUNG**

(21) Anmeldenummer: **88120587.6**

(22) Anmeldetag: **09.12.88**

(51) Int. Cl.⁴: **C02F 3/28**

(30) Priorität: **09.03.88 DE 3807607**

(43) Veröffentlichungstag der Anmeldung:
**13.09.89 Patentblatt 89/37**

(84) Benannte Vertragsstaaten:
**AT BE DE ES FR GB IT NL SE**

(71) Anmelder: **Passavant-Werke AG**

**D-6209 Aarbergen 7(DE)**

(72) Erfinder: **Berlenbach, Norbert, Dipl.-Ing.**
**Arndstrasse 45**
**D-6204 Taunusstein 1(DE)**

(54) Verfahren zur anaeroben Reinigung von Ab- und Prozesswasser.

(57) Verfahren zur anaeroben Reinigung von Ab- und Prozesswasser, bei dem $H_2S$-haltiges Biogas entsteht.

$H_2S$ wirkt im Biogas stark korrosiv. Bisher bekannte Verfahren zur Abreicherung des Biogases an $H_2S$ bedienen sich eines Oxidationsmittels, z.B. Eisenoxid. Die Regenerierung des Eisenoxids erfolgt mit (Luft)Sauerstoff. Die Dosierung des Sauerstoffs ist kritisch.

Gemäß der Erfindung wird die Aufgabe, ein unkritisches Verfahren zur $H_2S$-Abreicherung anzubieten, dadurch gelöst, daß man das in dem parallel betriebenen Reaktor abgereicherte Biogas im Kreislauf in die Anaerobstufe zurückführt und dort als Strippgas benutzt. Es ist dort weder eine pH-Wert-Einstellung noch die Benutzung von Fällmitteln noch eine zusätzliche Zufuhr von Sauerstoff erforderlich.

EP 0 331 806 A1

Verfahren zur anaeroben Reinigung von Ab- und Prozesswasser

Die Erfindung betrifft ein Verfahren zur anaeroben Reinigung von Ab- und Prozesswasser.

Verschiedene Ab- und Prozesswässer haben einen hohen Gehalt an schwefelhaltigen Verbindungen. Bei dem anaeroben Abbau dieser Verbindungen werden diese zu $H_2S$ reduziert, das in gelöster Form bei niedrigem pH-Wert des Abwassers toxisch auf die anaeroben Bakterien wirkt. Die ständige Zugabe von Lauge zur Anhebung des pH-Werts auf den Neutralpunkt ist teuer und nicht zu verantworten. Enthält das Biogas den Schwefelwasserstoff in größeren Mengen, dann muß das Biogas vor Verwertung zur Energiegewinnung von diesem korrosionsfördernden Bestandteil befreit werden.

So ist es z.B. bekannt, das Biogas durch Hindurchleiten durch eine Eisenoxid enthaltende Reinigungsmasse in einem parallel betriebenen Reaktor vom Schwefel-Wasserstoff zu befreien (DE-PS 11 40 662). Dabei entsteht Eisensulfid, das durch Zufuhr von Luft oder Sauerstoff zu Eisenoxid regeneriert wird. Beide Vorgänge läßt man vorzugsweise gleichzeitig ablaufen, indem dem zu entschwefelnden Biogas vor dem Auftreffen auf die Reinigungsmasse Sauerstoff zugesetzt wird.

Dieses Verfahren ist nicht ungefährlich. Da man mit einem Sauerstoffüberschuß arbeiten muß, kann ein explosionsfähiges Gasgemisch entstehen. Dosiert man den Sauerstoff dagegen niedriger, dann erschöpft sich die Reinigungsmasse frühzeitig, weil sie nicht mehr ausreichend regeneriert wird.

Es sind auch biologisch arbeitende Rieselbettreaktoren bekannt, in denen dem Biogas das $H_2S$ durch biologische Oxydation entzogen wird. Die Oxydationsprodukte werden durch die Befeuchtungsflüssigkeit aus dem Reaktor ausgeschleust (DE-OS 35 42 345).

Die Aufgabe, das $H_2S$ möglichst schon bei der Entstehung im Anaerobreaktor in der gelösten Phase zu eliminieren, wird gemäß der vorliegenden Erfindung dadurch gelöst, daß man einen Teil des in dem parallel betriebenen Reaktor von $H_2S$ abgereicherten Biogases im Kreislauf in die Anaerobstufe zurückführt und dort als Strippgas zum Eliminieren des gelösten $H_2S$ verwendet. Der parallel betriebene Reaktor kann auf biologische, physikalische oder chemische Art die Elimination vornehmen. Die Reaktionsprodukte des $H_2S$ werden getrennt aus dem Reaktor ausgeschleust, so daß sowohl der zurückgeführte als auch der zur Energiegewinnung abgeführte Teil des Biogases schadstoffarm ist. Die Ausschleusung erfolgt bei biologischen Reaktoren z.B. mit Hilfe des den biologischen Rasen befeuchtenden Waschwassers.

Vorzugsweise wälzt man mit dem rückgeführten Teil des Biogases den Inhalt des Anaerobreaktors um, so daß hierfür zusätzliche Mittel entbehrlich sind. Der Kontakt zwischen dem Strippgas und dem gelösten $H_2S$ wird durch dieses intensive Umwälzen sehr gefördert.

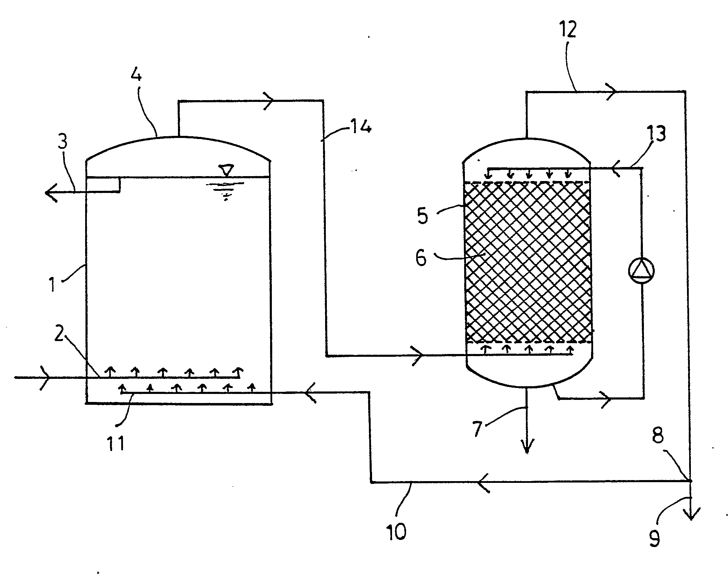

Die Zeichnung zeigt eine erfindungsgemäße Anlage zur Anaerobreinigung und $H_2S$-Abreicherung in schematischer Darstellung. Das zu reinigende Ab- bzw. Prozesswasser betritt den Anaerobreaktor 1 im unteren Behälterbereich über eine Verteilereinrichtung 2. Das an Schadstoffen, wie z.B. auch $H_2S$, abgereicherte Klarwasser wird im oberen Bereich abgezogen (3), während das Biogasgemisch im Behälterdom 4 abgezogen und über die Verbindungsleitung 14 in den Parallelreaktor 5 geleitet wird. Hier wird in einem von Waschwasser 13 oder einem Lösungsmittel im Gegenstrom durchflossenen Rieselbett 6 das $H_2S$ aus dem Biogas eliminiert und unten als Reaktionsprodukt (S oder $SO_4$) ausgeschleust (7). Das schadstofffreie Biogas 12 wird an der Verzweigstelle 8 aufgeteilt in einen der Energiegewinnung zugeführten Teilstrom 9 und einen weiteren Teilstrom 10, der in den Anaerobreaktor 1 zurückgeführt und dort über einen Verteiler 11 als Umwälzgas eingeblasen wird.

## Ansprüche

1. Verfahren zur anaeroben Reinigung von Ab- und Prozesswasser, bei dem $H_2S$-haltiges Biogas entsteht, das in einem parallel zum Anaerobreaktor betriebenen Reaktor behandelt und vom $H_2S$ weitgehend befreit wird, **dadurch gekennzeichnet,** daß das Biogas nach der $H_2S$-Abreicherung teilweise in den Anaerobreaktor zurückgeführt wird und dort im wesentlichen das in gelöster Form im Abwasser enthaltene $H_2S$ aufnimmt und in den parallel betriebenen Reaktor überführt, und daß die Reaktionprodukte der $H_2S$-Abreicherung separat aus dem parallel betriebenen Reaktor ausgeschleust werden.

2. Verfahren nach Anspruch 1, **dadurch gekennzeichnet,** daß man mit dem rückgeführten Teil des Biogases gleichzeitig den Inhalt des Anaerobreaktors umwälzt und dadurch einen intensiven Kontakt zum gelösten $H_2S$ herstellt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 461 684 (DEGREMONT) * Seite 6, Ansprüche 1-3; Seite 3, Zeile 26 - Seite 4, Zeile 5 * --- | 1,2 | C 02 F 3/28 |
| X | FR-A-2 484 990 (DEGREMONT) * Seite 5, Anspruch 1; Seite 3, Zeile 25 - Seite 4, Zeile 4 * ----- | 1,2 | |

|  |
|---|
| RECHERCHIERTE SACHGEBIETE (Int. Cl.4) |
| C 02 F |

Der vorliegende Recherchenbericht wurde für alle Patentansprüche erstellt

| Recherchenort | Abschlußdatum der Recherche | Prüfer |
|---|---|---|
| DEN HAAG | 12-06-1989 | TEPLY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
········································································
& : Mitglied der gleichen Patentfamilie, übereinstimmendes Dokument

EPO FORM 1503 03.82 (P0403)